(19) Europäisches Patentamt
European Patent Office
Office européen des brevets

(11) Publication number: **0 009 285**
**B2**

(12) **NEW EUROPEAN PATENT SPECIFICATION**

(45) Date of publication of the new patent specification: **19.12.84**

(51) Int. Cl.[3]: **C 07 C 59/68,** C 07 C 51/367, A 01 N 39/02

(21) Application number: **79200507.6**

(22) Date of filing: **14.09.79**

# (54) Process for the preparation of optically active 2-phenoxy propionic acids and herbicidal preparations containing such acids.

(30) Priority: **19.09.78 NL 7809514**

(43) Date of publication of application: **02.04.80 Bulletin 80/07**

(45) Publication of the grant of the patent: **19.12.84 Bulletin 84/51**

(45) Mention of the opposition decision: **12.05.82 Bulletin 82/19**

(84) Designated Contracting States: **AT DE FR GB IT NL**

(56) References cited:
**FR-A-2 371 414**
**GB-A-1 114 040**
**US-A-2 723 993**

**Chambers dictionary of science and technology, vol. 1, p. 64**

**Glossary of Chemical Terms, page 23**

(73) Proprietor: **AKZO N.V.**
**Velperweg 76**
**NL-6824 BM Arnhem (NL)**

(72) Inventor: **Eveleens, Willem**
**Boerhavelaan 122**
**NL-7555 BE Hengelo (NL)**
Inventor: **Spaans, Johannes**
**Marellenkamp 5**
**NL-7576 GD Oldenzaal (NL)**
Inventor: **Wissink, Hendrik Gerrit**
**Catsstraat 11**
**NL-7471 XP Goor (NL)**

(74) Representative: **Sieders, René et al**
**P.O. Box 314**
**NL-6800 AH Arnhem (NL)**

(56) References cited:
**CRC Handbook of Chemistry and Physics, 53rd edn. pages D 181, 189**

**EP 0 009 285 B2**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The invention relates to a process for the preparation of 2-phenoxy propionic acids, having predominantly the D-isomer structure, of the general formula

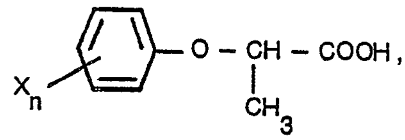

where X represents halogen atoms and/or methyl groups and n is an integer from 1 to 5, by condensing an alkalimetal salt of 2-chloropropionic acid which is dextrorotatory in water with an alkalimetal derivative of a phenol of the general formula

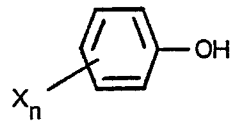

A process of the type indicated above is described in the British Patent 1 114 040.

To this known process, however, although it offers the important advantage of selective formation of the desired optical isomer with its herbicidal effect, there is the drawback that the condensation reaction must be carried out in an organic solvent, such as toluene or xylene. This complicates carrying out the process on a technical scale and provisions need be made for recovering the solvent. Moreover, the use of such solvents is little attractive from an environmental point of view.

It has now been found that selective formation of the desired isomer also can be effected in an aqueous medium, provided that the water content of the reaction mixture is kept sufficiently low.

According to the invention the condensation is carried out in an aqueous medium in such a way that the overall amount of water in the reactor mixture is not more than 8 moles per mole of alkali metal salt of 2-chloropropionic acid.

From the results of experiments it appears that in water the condensation is as a rule little selective and generally yields a racemic product. The selectivity, however, increases with decreasing amount of water, so that the endproduct will gradually contain more of the desired D-2-phenoxy propionic acids.

For practical reasons the resulting 2-phenoxy propionic acid product will have to contain more than about 85% of the active D-form for the preparation to be commercially attractive.

Correspondingly, if per mole of 2-chloropropionic acid not more than 8 moles of water are present according to the invention, the product obtained will normally satisfy that condition and oftentimes contains at least about 89% of the active D-form.

It is preferred that the reaction should be carried out in the presence of fewer than 3 moles of water per mole of 2 chloropropionic acid, as a result of which the phenoxy product will on an average contain 93% or more of the D-form.

The reaction can, of course, not be carried out water-free and the lower limit of the amount of water used is determined by the manageableness of the reaction mixture. Whether in the presence of little water the reaction mixture is still in solution or in the form of a slurry is partly dependent on the temperature. The reaction is usually carried out at elevated temperature, and preferably at 80°C or higher. As mentioned in the British Patent 1 114 040, the optically active starting material, namely the dextrorotatory alkali metal salt of 2-chloropropionic acid is obtained by hydrolysis of the laevo-rotatory methyl ester of the propionic acid. This hydrolysis may be carried out in situ or separately. It is preferred that the ester should be hydrolysed separately and the resulting propionic salt, which may be purified of methanol and dried, be added to the phenol derivative, with due observation according to the invention of the maximum permissible amount of water in the reaction mixture. By this route the process also may very well be carried out continuously. The invention is further described in the following examples.

Example 1

In five experiments D-2-(2-methyl 4-chlorophenoxy) propionic acid was prepared at various water contents in the reaction mixture. In the first four experiments use was made of the following procedure.

To a mixture of 122,5 g of L-2-chloropropionic methyl ester (actual $\alpha_D^{20} = -25{,}2°$) and 8 g of methanol there were added over a period of 40 minutes, with stirring and at a temperature below 40°C, 133 g of 33%-sodium hydroxide solution.

Subsequently, over a period of 60 minutes the resulting mixture was added, with stirring, to a mixture of 143 g of 2-methyl 4-chlorophenol, 38 g of sodium hydroxide and x g of water, with the

temperature being kept at 90°C. The total mixture was then stirred for 1 more hour at 90°C.

The amount of x was determined by adding the sodium hydroxide as 24%, 30%, 50% and 60% aqueous solution, respectively in Experiments 1 to 4

Next, the reaction mixture was neutralized with hydrochloric acid to pH = 7 and subsequently extracted with three times 50 ml of xylene to remove the excess chlorocresol.

The aqueous solution was acidified then with hydrochloric acid to pH = 4, which was attended with the separation of an oily phase. This oil was extracted with ether, after which the extract was freed from ether by evaporation and dried to constant weight.

In Experiment 5 the following procedure was used.

To a mixture of 122,5 g of L-2-chloropropionic methyl ester and 8 g of methanol there were added over a period of 40 minutes, with stirring and at a temperature below 40°C, 88 g of 50%-sodium hydroxide solution. This mixture was subsequently purified and dried at 40°C and 20 mm mercury pressure. The product obtained consisted of 130 g of dry sodium salt of L-2-chloropropionic acid. Next, over a period of 60 minutes this dry salt was added, with stirring and at 90°C, to a mixture of 143 g of 2-methyl 4-chlorophenol, 38 g of sodium hydroxide and 20 g of water. For the rest the same procedure was used as described above.

The results of these experiments are summarized in the following table.

| | Reaction mixture | | Phenoxy endproduct | |
|---|---|---|---|---|
| Exp. | Total water content (g) | Molar ratio $H_2O$/propionate | $\alpha_D^{20}$ (in acetone) | % D-isomer |
| 11 | 228 | 12,7 | + 18,9 | 83,6 |
| 2 | 196 | 10,9 | + 21,0 | 87,3 |
| 3 | 145 | 8,1 | + 22,1 | 89,2 |
| 4 | 132 | 7,3 | + 22,4 | 89,8 |
| 5 | 38 | 2,1 | + 25,0 | 94,4 |

The percentage D-isomer in the endproduct was calculated from the measured rotation $\alpha_D^{20}$ and the rotation $\alpha_D^{20} = -28,15°$ (10% in acetone) which holds for pure D-2-(2-methyl 4-chlorophenoxy) propionic acid. The table shows that the percentage of the desired D-isomer in the endproduct increases with decreasing molar ratio water/propionate in the reaction mixture, i.e. to such an extent that of from a molar ratio of about 8 it exceeds 89%.

### Example II

In a further five experiments the influence of water was tested in the preparation of D-2-(2,4-dichlorophenoxy) propionic acid.

Experiments 7 and 8 were carried out using the same procedure as in Example I for Experiments 1—4. The saponified propionic ester was added to a caustic mixture of 163 g of 2,4-dichlorophenol, the amount of x being determined by employing 48% and 60% caustic solution, respectively in Experiments 7 and 8.

In the Experiments 6, 9 and 10 the procedure of Experiment 5 of Example I was followed using a different batch of the propionic ester starting material (actual $\alpha_D^{22} = -25,5°$).

The purified and dried sodium salt obtained from this material was added to a mixture of 163 g of 2,4-dichlorophenol, 38 g of sodium hydroxide and an amount of water equal to 220 g, 95 g and 31 g, respectively in Experiments 6, 9 and 10.

The relevant experimental results are summarized in the next table.

| | Reaction mixture | | Phenoxy endproduct | |
|---|---|---|---|---|
| Exp. | Total water content (g) | Molar ratio $H_2O$/propionate | $\alpha_D^{20}$ (in chloroform) | % D-isomer |
| 6 | 223 | 13,2 | + 9,3 | 74,0 |
| 7 | 148 | 8,2 | + 12,9 | 83,7 |
| 8 | 132 | 7,3 | + 13,6 | 85,3 |
| 9 | 113 | 6,3 | + 13,9 | 86,1 |
| 10 | 49 | 2,7 | + 16,7 | 93,4 |

The percentage D-isomer was calculated using $\alpha_D^{20} = +19{,}3°$ (in chloroform for pure D-2-(2,4-dichlorophenoxy) propionic acid.

Again it is seen from the table that at molar ratios below about 8 the percentage D-isomer in the endproduct exceeds the 85 level.

Example III

Using the procedure of Experiments 1—4 of Example I a single preparation of D-2-(2-methylphenoxy) propionic acid was carried out. To this end the saponified propionic ester was added to a mixture of 108 g of 2-methyl phenol, 38 g of sodium hydroxide and 18 g of water.

Thus, in the reaction mixture the total water content amounted to 125 g, giving a molar ratio water to propionate of 6.9.

The rotation $\alpha_D^{20}$ of the endproduct was determined at +15,6° by measurement in chloroform. Using $\alpha_D^{20} = +18{,}2°$ (in chloroform) for pure D-2-(2-methylphenoxy) propionic acid the optical purity of the endproduct was calculated at 93% of the D-isomer.

**Claims**

1. A process for the preparation of 2-phenoxy propionic acids, having predominantly the D-isomer structure of the general formula

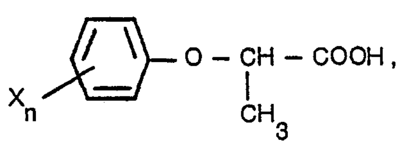

where X represents halogen atoms and/or methyl groups and n is an integer from 1 to 5, by condensing an alkali metal salt of 2-chloropropionic acid which is dextrorotatory in water with an alkali derivative of a phenol of the general formula

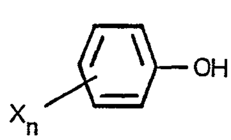

at elevated temperature, and acidifying the resultant alkali metal salt of the 2-phenoxy propionic acid to yield the corresponding acid, characterized in that the condensation is carried out in an aqueous medium, care being taken that the overall amount of water in the reaction mixture is not more than 8 moles per mole of alkali metal salt of 2-chloropropionic acid.

2. A process according to claim 1, characterized in that per mole of propionate fewer than 3 moles of water are present.

**Patentansprüche**

1. Verfahren zur Hestellung von 2-Phenoxypropionsäuren mit vorwiegend einer D-Isomer-Struktur und mit der allgemeinen Formel

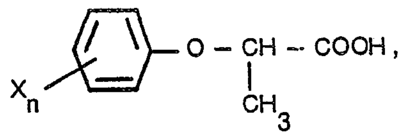

wobei X Halogenatome und/oder Methylgruppen darstellt und n eine ganze Zahl von 1 bis 5 ist, durch Kondensation eines in Wasser rechtsdrehenden Alkalimetallsalzes der 2-Chlorpropionsäure mit einem Alkaliderivat eines Phenols der allgemeinen Formel

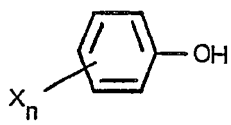

bei erhöhter Temperatur und Ansäuerung des erhaltenen Alkalimetallsalzes der 2-Phenoxypropionsäure, wobei die entsprechende Säure entsteht, dadurch gekennzeichnet, dass die Kondensation in einem wässrigen Medium erfolgt, wobei darauf zu achten ist, das die Gesamtmenge an Wasser in der Reaktionsmischung nicht mehr als 8 Mol pro Mol Alkalimetallsalz der 2-Chlorpropionsäure beträgt.

2. Verfahren gemäss Anspruch 1, dadurch gekennzeichnet, dass pro Mol Propionat weniger als 3 Mol Wasser anwesend sind.

**Revendications**

1. Procédé pour préparer des acides 2-phénoxypropioniques ayant essentiellement la structure D-isomère de la formule générale

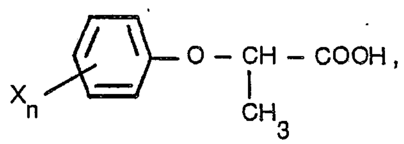

où X représente des atomes d'halogène et/ou des groupes méthyles et n un nombre entier de 1 à 5, par condensation d'un sel alcalin d'acide 2-chloropropionique qui est dextrogyre dans l'eau avec un derivé alcalin d'un phénol de la formule générale

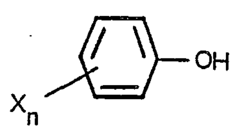

à température élevée, et acidification du sel alcalin formé ainsi de l'acide 2-phénoxypropionique en vue de l'obtention de l'acide correspondant, caractérisé en ce que la condensation est effectuée dans un milieu aqueux de telle manière que la quantité totale d'eau dans le mélange réactionnel n'est pas supérieure à 8 moles par mole de cel alcalin d'acide 2-chloropropionique.

2. Procédé selon la revendication 1, caractérisé en ce que la quantité présente d'eau est de moins de 3 moles par mole de propionate.